# EUROPEAN PATENT APPLICATION

(11) **EP 4 311 494 A1**
(43) Date of publication of application: **31.01.2024**
(21) Application number: 22187368.0
(22) Date of filing: 28.07.2022
(51) Int. Cl.: A61B 6/00, A61B 6/03

(54) **A CT SCANNER AND A SCANNING METHOD FOR PERFORMING A BRAIN SCAN**

(71) Applicant: Koninklijke Philips N.V., 5656 AG Eindhoven (NL)
(72) Inventor: BUELOW, Thomas, Eindhoven (NL); HARDER, Tim Philipp, Eindhoven (NL); SENEGAS, Julien Thomas, 5656AG Eindhoven (NL); DESHPANDE, Hrishikesh Narayanrao, 5656AG Eindhoven (NL); FRERKING, Lena Christina, Eindhoven (NL); GOTMAN, Shlomo, Eindhoven (NL); KRUEGER, Sascha, Eindhoven (NL); COETSER, Edna, Eindhoven (NL)
(74) Representative: Philips Intellectual Property & Standards

(57) **Abstract**

A CT scanner for performing a brain scan performs a low dose 3D scan with a first field of view. By analyzing the low dose 3D scan, for example using landmark detection, a proposed field of view is obtained for a full dose 3D brain scan. A representation of the proposed field of view is displayed, and a final field of view is generated, as the proposed field of view or an updated version of the proposed field of view provided by a user of the CT scanner.

## Description

### FIELD OF THE INVENTION

The present invention relates to a CT scanner and scanning method for performing a brain scan.

### BACKGROUND OF THE INVENTION

Medical imaging, such as CT imaging, is an increasingly important tool in correct analysis and assessment of a subject/patient.

Proper patient positioning is one of the most important considerations in medical images, particularly CT imaging, to ensure a high-quality scan. In CT imaging of the head ("head CT"), quality relates to the diagnostic aspect of the scan, e.g. diagnostic usefulness, as well as the dose exposure to the patient, which is preferably minimized or reduced.

When performing a CT scan of the human brain, it is the operator's task to position the patient and to plan the scan such that the entire brain is within the field of view of the scan while at the same time the eyes are optimally spared the direct radiation. Some CT scanners allow the gantry of the CT system to be tilted which can support the optimal scanning of the patient, especially in situations where the patient's condition does not allow to tilt the head to the required orientation.

Another task is to assure that the resulting scan is reformatted to a standard view which makes it easier for the radiologist to read the exam. Official standards for medical imaging techniques, such as head CT, provide guidelines for appropriate medical scanning, e.g. to generate clinically useful and repeatable medical images. Failure to adhere to the guidelines reduces a quality of images produced by the scanning procedure and can lead to unnecessary radiation exposure to at-risk organs or anatomical features, such as the eye lenses. Similarly, compliance with the guidelines produces an image of high quality, as the guidelines will typically ensure that images contain diagnostically sensitive or useful structures.

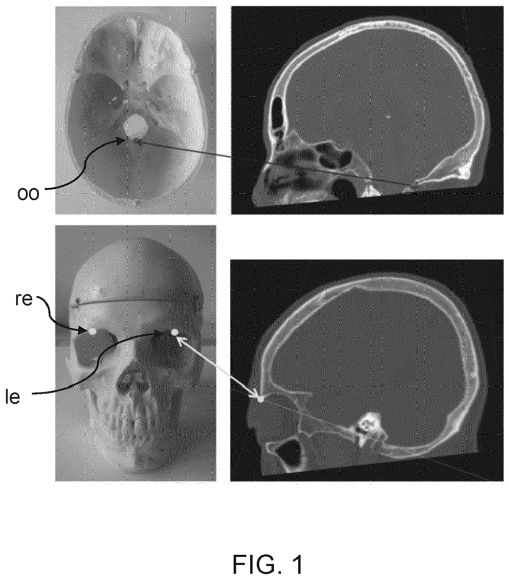

These guidelines are usually defined with regard to a set of anatomical landmarks. For instance, one set of guidelines for head CT, published by the American Association of Physicists in Medicine, defines a recommended scan angle as "parallel to a line created by the supraorbital ridge and the inner table of the posterior margin of the foramen magnum". Figure 1 for example illustrates the position of the opisthion of the occipital bone (oo) (which is the median point of the posterior margin of the foramen magnum), and the points on the supra-orbital ridge of the left (le) and the right eye (re) with respect to the skull, and corresponding CT images of the subject.

Planning of a CT scan of the brain in particular is challenging because there is a small margin between cropping of the inferior part of the brain and including the orbits of the eyes within the field of view. Neither of these should happen in an optimal scan, but in order to assure full brain coverage, most technologists would add a margin to the field of view and accept partial or full irradiation of the eyes during scan planning (in violation of the guidelines). The situation is complicated by the fact that currently the scan planning is done based on 2D surview scans (usually lateral projections).

There is therefore a need for an improved scan procedure which enables the radiation exposure of the eyes to be reduced during a brain scan.

### SUMMARY OF THE INVENTION

The invention is defined by the claims.

According to examples in accordance with an aspect of the invention, there is provided a CT scanner for performing a brain scan, comprising:
a gantry;
an X-ray tube and detector within the gantry;
a display; and
a controller, wherein the controller is configured to:
   control the CT scanner to perform a low dose 3D scan with a first field of view;
   analyze the results of the low dose 3D scan to derive a proposed field of view for a full dose 3D brain scan;
   display a representation of the proposed field of view; and
   determine a final field of view as the proposed field of view or an updated version of the proposed field of view provided by a user of the CT scanner.

This scanner enables a low dose 3D scan (i.e. a so-called scout scan or surview scan) to be used to adjust the field of view for an eventual full/regular dose scan. The low dose 3D scan enables the field of view to be adjusted so that the desired brain area is covered without radiating the eyes.

The system performs automatic analysis of the low dose scan to provide a recommended field of view, which can be accepted by the operator or modified. The recommended field of view is the result of a particular patient position, gantry position and scan plan.

The availability of a 3D low dose scan (to create a 3D surview image) enables anatomic landmarks to be detected in this low dose 3D image so that the head orientation can be determined. The full 3D orientation of the head can be determined, not just the rotation about one axis as can be achieved with a lateral 2D surview image.

The low dose for example is achieved with an X-ray tube current of around 2mAs and a corresponding radiation intensity CTDI-vol of around 0.2mGy. The full/regular dose for example is achieved with an X-ray tube current of around 300mAs and a corresponding radiation intensity CTDI-vol of around 50 to 60mGy. Thus, the low dose has a tube current for example in the range 0.2 to 5% of the full/regular dose and a radiation intensity in the range 0.1% to 2% of the full/regular dose.

In addition to avoiding radiation of the eyes, the analysis may also be used (e.g. based on AI) to detect foreign metal objects. The gantry can then be tilted or the plane of the axial slice reconstruction can be adapted in such a way that the foreign object is avoided in the given axial field-of-view.

The proposed field of view is part of an overall scan plan. The scan plan comprises a definition of the scan length which is defined by the initial and final table positions. It also includes a definition of a box around the target anatomy defining the field of view used for reconstruction.

The first field of view for example includes the brain and the eyes and the final field of view excludes the eyes. Thus, the low dose planning is used when the eyes are (e.g. partially) within the field of view, but the final field of view excludes the eyes.

The controller is for example configured to determine a final field of view by setting an angle for the gantry tilt. For a scanner with adjustable gantry angle, this is one of the parameters that can be selected based on the analysis of the low dose scan. The gantry tilt in such cases may also be considered, for the purposes of this application, to be part of the scan plan.

If the scanner is not equipped with gantry tilt functionality, or if the patient position (head orientation) does not allow to fix the problem of the eye being in the field of view even with gantry tilt, there could be an alternative solution, to alert the operator to reposition the patient's head.

The controller is for example configured to analyze the results of the low dose 3D scan to identify landmarks on the skull. These landmarks can be used to identify the locations of the different regions of the head within the initial field of view and to determine the orientation of the head. Image detection methods for detection of landmarks on the skull (using a regular dose) are known.

The landmarks may comprise the supra-orbital margins of the left and right eye, the occipital bone position and a top of the skull.

The supra-orbital margins and the occipital bone enable the head orientation and position to be determined. A plane defined by these landmarks establishes the lowest axial slice of an optimally planned brain scan. The top of the skull enables the required limit of the field of view to be determined.

The controller is for example configured to derive a proposed field of view in the form of a rectangular 3D box which encloses the brain. This box aims to avoid irradiation of the eyes.

The controller is configured to derive a rectangular 3D box as:
a box with axes parallel to the CT scanner coordinate system; or
a box tilted about one axis compared to a box with axes parallel to the CT scanner coordinate system.

These different box definitions are for example suitable for different scanners, for example with or without the ability provide a gantry tilt.

The controller may be configured to freely rotate the box in 3D space compared to a box with axes parallel to the CT scanner coordinate system to generate a diagnostic standard view representation. A box freely rotated in 3D space can for example be used to provide a rectified representation of the scan as preferred by a radiologist for diagnostic reading.

The controller is for example further configured to generate images of axial superior and inferior slices for the final field of view from the low dose 3D scan.

These images, derived from the low dose scan, typically will not show enough information for diagnostic purposes. However, they show the coarse anatomy quite well, given the high contrast between bone and soft tissue. A user may for example look at these images to verify that the anatomy is included as expected by the radiologist. The axial view is the common representation used for diagnostic reading.

The scanner may further comprise a camera system for detecting a head position and orientation of the patient relative to the CT scanner, wherein the controller is further configured to derive the first field of view from the detected head position and orientation.

In this way, the first field of view for the low dose can already aim to exclude the eyes, by using a camera-based position detection system.

The invention also provides a method of preparing a patient for a CT brain scan, comprising:
controlling a CT scanner to perform a low dose 3D scan with a first field of view;
analyzing the results of the low dose 3D scan to derive a proposed field of view for a full dose 3D brain scan;
displaying the proposed field of view to a user of the CT system; and
determining a final field of view as the proposed field of view or as an updated version of the proposed field of view provided by the user.

The method may further comprise analyzing the results of the low dose 3D scan to identify landmarks on the skull, such as the supra-orbital margins of the left and right eye, the occipital bone position and a top of the skull.

The method may comprise deriving a proposed field of view in the form of a rectangular 3D box which encloses the brain, and the method comprises deriving a rectangular 3D box as:
a box with axes parallel to the CT scanner coordinate system; or
a box tilted about one axis compared to a box with axes parallel to the CT scanner coordinate system.

The method may comprise freely rotating the box in 3D space compared to a box with axes parallel to the CT scanner coordinate system to generate a diagnostic standard view representation.

The method may comprise controlling a camera system to detect a head position and orientation of the patient relative to the CT scanner and the controller is further configured to derive the first field of view from the detected head position and orientation.

The invention also provides a computer program comprising computer program code which is adapted, when said program is run on a computer, to implement the method defined above.

Another concept is a CT scanner for performing a brain scan, comprising:
a gantry;
an X-ray tube and detector within the gantry;
a camera system for detecting a head position and orientation of the patient relative to the CT scanner;
a display; and
a controller, wherein the controller is configured to:
   analyze the head position and orientation to determine a required field of view and/or patient head movement that may be required; and
   output a representation of the required field of view and/or required patient head movement to a user using the display.

The camera system is for example an overhead 2D or 3D (depth) camera mounted above the patient couch or one or more cameras integrated with the gantry. It is used to predict the desired tilt of the head and guide the operator in the optimal exam preparation. This can be achieved by tilting the CT gantry or by guiding the operator to orient the head of the patient according to the computed tilt. In both cases, the brain plane and CT gantry axes become parallel and the field of view for a brain scan can be determined in a way that it excludes the eyes for both the low dose scan and the actual scan or even avoids the need for a low dose scan at all.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows the locations of the opisthion of the occipital bone, and the points each on the supra-orbital ridge of the left and the right eye;
Figure 2 schematically illustrates a known CT system;
Figure 3 shows a method of preparing a patient for a CT brain scan, for example using the system of Figure 2;
Figure 4a shows a field of view box with axes parallel to the scanner coordinate system;
Figure 4b shows a field of view box which has been tilted about one axis to optimize the tight enclosure of the brain;
Figure 4c shows the field of view box freely rotated in 3D space to optimally capture the brain anatomy;
Figure 5 shows visual representations of the scan plan;
Figure 6 shows examples of axial slices derived from the 3D low dose scan; and
Figures 7a and 7b show images of the head of an exemplary subject acquired with an overhead camera with respective landmarks marking the eyebrows, eyes, nose, lips and the outer shape of the face.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a CT scanner and method for performing a brain scan. A low dose 3D scan is performed with a first field of view. By analyzing the low dose 3D scan, for example using landmark detection, a proposed field of view is obtained for a full (i.e. regular) dose 3D brain scan. A representation of the proposed field of view is displayed, and a final field of view is generated, as the proposed field of view or an updated version of the proposed field of view provided by a user of the CT scanner.

The invention thus provides automatic analysis of the head anatomy from a low dose the 3D scan, to result in an automatic scan planning, preferably based on a proposed field of view in the form of a 3D scan box. For a CT scanner with gantry tilt control, the proposed scan plan includes guidance on the gantry tilt angle of the CT system for optimal scanning.

The basic known components of a CT scanner will first be described, before the modified functionality provided by the invention is explained.

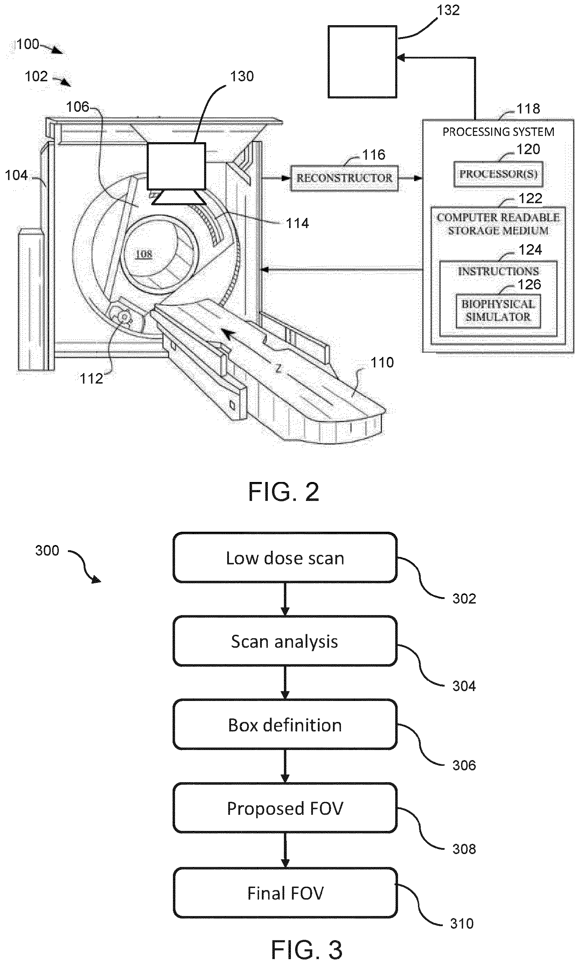

Figure 2 schematically illustrates a known CT system 100 including a CT scanner 102. The CT scanner 102 includes a generally stationary gantry 104 and a rotating gantry 106, which is rotatably supported by the stationary gantry 104 and rotates around an examination region 108 about a z-axis. A subject support 110, such as a couch, supports an object or subject in the examination region 108.

A radiation source 112, such as an x-ray tube, is rotatably supported by the rotating gantry 106, rotates with the rotating gantry 106, and emits radiation that traverses the examination region 108.

A radiation sensitive detector array 114 subtends an angular arc opposite the radiation source 112 across the examination region 108. The radiation sensitive detector array 114 detects radiation traversing the examination region 108 and generates an electrical signal(s) (projection data) indicative thereof.

The detector array 114 can include single layer detectors, direct conversion photon counting detectors, and/or multi-layer detectors. The direct conversion photon counting detectors may include a conversion material such as CdTe, CdZnTe, Si, Ge, GaAs, or other direct conversion material. An example of multi-layer detector includes a double decker detector such as the double decker detector described in US patent 7,968,853 B2, filed April 10, 2006, and entitled "Double Decker Detector for Spectral CT,".

A reconstructor 116 of the imaging system 102 receives projection data from the detector array 114 and reconstructs one or more CT images from the projection data. The reconstructed CT images may comprise one or more 2D or 3D images. Mechanisms for reconstructing one or more CT images from projection data are well-established in the art.

A controller 118 is configured to process CT images generated by the imaging system 102. The controller 118 may include a processor 120 (e.g., a microprocessor, a controller, a central processing unit, etc.) and a computer readable storage medium 122, which excludes non-transitory medium, and includes transitory medium such as a physical memory device, etc.

The computer readable storage medium 122 may include instructions 124 for processing images as explained below, including detecting landmarks in a low dose (surview) scan and performing scan planning. The processor 120 is configured to execute the instructions 124. The processor 120 may additionally be configured to execute one or more computer readable instructions carried by a carrier wave, a signal and/or other transitory medium.

However, instead of a processor 120 executing instructions to perform a herein described method, the processor may instead comprise fixed-function circuitry (e.g. appropriately programmed FPGAs or the like) to carry out the described methods.

In some examples, the controller may also serve as an operator console. The controller 118 includes a human readable output device such as a monitor and an input device such as a keyboard, mouse, etc. Software resident on the controller 118 allows the operator to interact with and/or operate the scanner 102 via a graphical user interface (GUI) or otherwise.

In a variation, a separate controller (not shown) may serve as an operator console, and comprise the relevant operator console elements previously described.

Figure 2 also shows a camera 130 (discussed below) and a display 132 for presenting scan results, and well as scan planning results (so-called surview or scout scan results) to the operator.

Figure 3 shows a method 300 of preparing a patient for a CT brain scan, for example using the system of Figure 2.

The first step in the application of this invention is the acquisition of a low dose (or ultra-low dose) 3D scan of the patient in step 302. This is performed with a first field of view. The first field of view may be permitted to include irradiation of the eyes of the patient due to the ultra-low dose, but as explained below, measures may be taken to prevent even the low dose view irradiating the patient's eyes.

A low dose for example is achieved with an X-ray tube current of around 2mAs and a corresponding radiation intensity CTDI-vol of around 0.2mGy. A full/regular dose for example is achieved with an X-ray tube current of around 300mAs and a corresponding radiation intensity CTDI-vol of around 50 to 60mGy. Thus, the low dose has a tube current for example in the range 0.2 to 5% of the full dose and a radiation intensity in the range 0.1% to 2% of the full dose.

In step 304, the results of the low dose 3D scan are analyzed, in particular to derive a proposed field of view for a full dose 3D brain scan. The analysis of the low-dose scan enables the head anatomy to be analyzed. This can be done by various known medical image analysis techniques. One example is the detection of landmarks on the skull such as shown in Figure 1 (supra-orbital margins of the left and right eye and the opisthion of the occipital bone).

The analysis in step 304 for example involves the detection of these three landmarks. This is sufficient for the analysis of the 3D rotation state of the head.

Examples of approaches for the landmark detection may for example be found in Hrishikesh Deshpande, Axel Saalbach, Tim Harder, Stewart Young, Thomas Buelow, "Deep learning for the detection of landmarks in head CT images and automatic quality assessment," Proc. SPIE 11596, Medical Imaging 2021: Image Processing, 115960N (15 February 2021); https://doi.org/10.1117/12.2581810. Landmark detection is also described in WO2022/023228.

A plane defined by these three landmarks establishes the lowest axial slice of an optimally planned brain scan. In order to define a scan plan, an additional landmark at the top of the skull is preferably also detected in step 304.

Based on the derived head anatomy, a rectangular 3D box enclosing the brain is then derived in step 306. This rectangular box may be considered to represent a proposed field of view for the eventual full dose scan.

The proposed field of view is displayed to a user of the CT system in step 306. In this way, based on the scan box and the 3D low dose scan, the operator receives a visual representation of the scan plan in step 308. This visual representation illustrates the expected scan results when scanning is performed with the current patient position, gantry tilt (if it is adjustable) and other parameters of the scan plan defining the field of view (in the form of a scan box). The expected results are presented in real time, so that the user can manipulate the scan box. This manipulation may be achieved by a graphical user interface.

A final field of view is then derived in step 310. It may be the proposed field of view (if approved by the user) or it may be an updated version of the proposed field of view provided by the user.

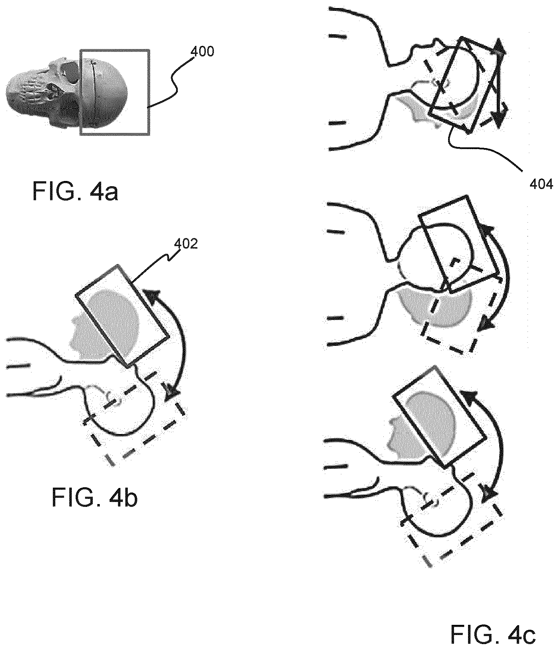

As explained above, a rectangular box is derived as the field of view. Two different box types can be derived as illustrated in Figure 4.

Figure 4a shows a box 400 with axes parallel to the scanner coordinate system. Such a box can be proposed for systems that do not allow for a tilt of the gantry.

Figure 4b shows a box 402 which has been tilted about one axis to optimize the tight enclosure of the brain. The single degree of freedom is chosen to correspond to the gantry tilt angle. It corresponds to the patient's nodding angle. It can be proposed for systems that do allow for a gantry tilt. The tilt angle between the scan boxes 400 and 402 represents the proposed gantry tilt to be applied to the scanner hardware.

The rectangular box can also be manipulated to result in a standard view for diagnostic purposes. Figure 4c shows a rectangular box 404 which has been freely rotated in 3D space to optimally capture the brain anatomy. This box represents the rectified representation of the final scan result as preferred by the radiologist for diagnostic reading.

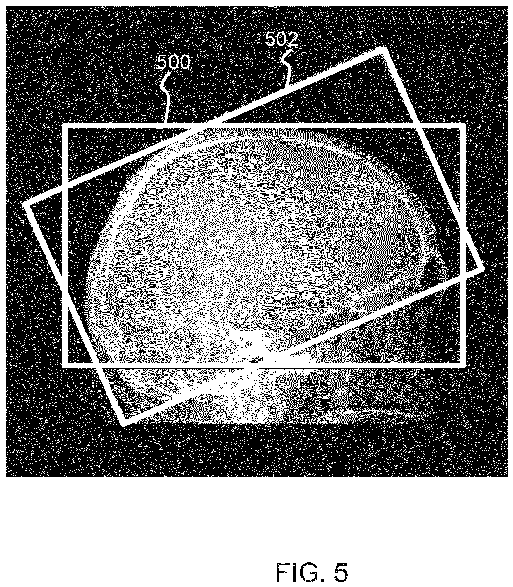

Figure 5 shows the visual representations of the scan plan provided in step 308.

Box 500 (not tilted) and box 502 (tilted) are overlaid on a 2D synthetic lateral scout scan. The synthetic scout scan is derived from the 3D low dose scan. Starting from these scan box representations, the operator can decide whether a gantry tilt should be applied or not. Also, the start and end points (superior and inferior positions of the scan box) can be adapted by the user.

In addition to the visual scan box representation shown in Figure 5, the 3D low dose scan allows generation of axial slices representing the superior and inferior slice of the expected final scan result based on the choices made by the operator.

These results can be adapted to the operator's choice of scanning without gantry tilt (box 400), with gantry tilt (box 402) and also shown in the reformatted way that will be transferred to the radiologist (box 404).

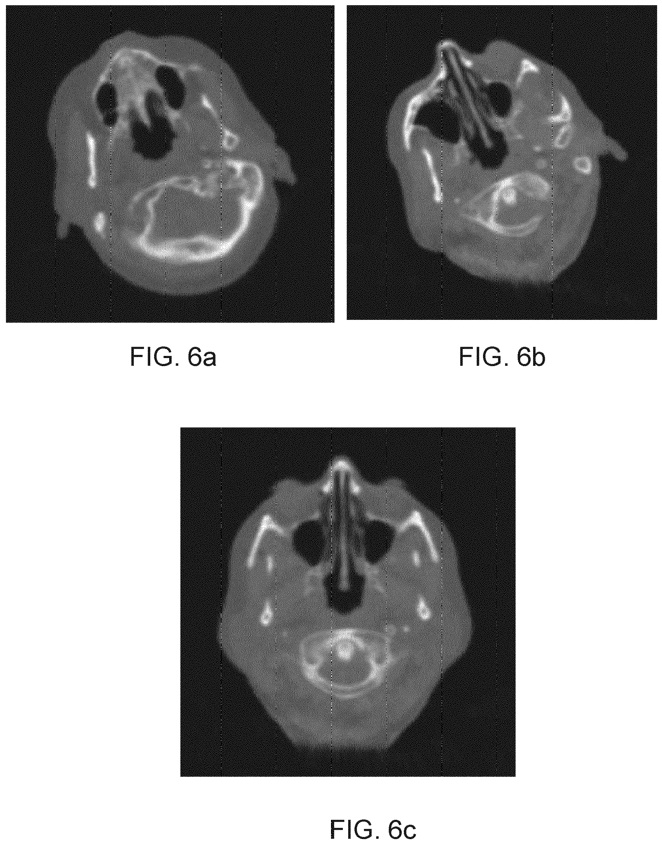

Figure 6 shows examples of axial slices derived from the 3D low dose scan. Figure 6a shows simulated results of a scan performed according to scan box 400, Figure 6b shows simulated results of a scan performed with tilted gantry according to scan box 402 and Figure 6c shows simulated results of a scan reformatted according to scan box 404.

Anatomically aligned reformatting of the scan is clinically important, for diagnostic reading. Measures such as the ASPECT score for the fast assessment of severity of a stroke rely on symmetric representation of the brain anatomy. Manual reformatting costs the radiologist valuable time.

As explained above, the most accurate way to align the brain plane and gantry is to tilt the gantry so that its axis is parallel to the head tilt measured during the 3D low dose scan. However, not all CT devices offer the possibility to tilt the gantry. Without this feature, the only chance to align the brain plane and gantry is to manually change the position of the head, e.g. by using cushions or different kinds of head supports.

This manual procedure is done mainly by eye, with no direct feedback on the correct alignment, and most of the time before the acquisition of the low dose scan. To be sure the new head tilt actually fits the gantry axis, an additional low dose scan is for example necessary, which would again expose radiation to the entire head including the eyes.

In order to prevent that even the low dose scan irradiates the eyes, a modification makes use of a camera system to determine the field of view of the low dose scan. In particular, an overhead depth camera, schematically shown as 130 in Figure 2, may be mounted above the patient couch to predict the desired tilt of the head and guide the operator in the optimal exam preparation.

This can be achieved by tilting the CT gantry if possible or by guiding the operator to orient the head of the patient according to the computed tilt. In both cases, the brain plane and CT gantry axes become parallel and the field of view for a brain scan can be determined in a way that it excludes the eyes for both the low dose scan and the actual scan.

A depth map gathered by the depth camera gives information about the height of every single part of the body. By using landmark detection algorithms, which usually work on color, grey, depth, or IR images, it is possible to detect specific surface-parts of the face, e.g. different points on the eyebrows, the nose, the lips, the chin etc..

Standard 3D cameras usually offer the possibility to acquire both color and depth images at the same time. With a proper calibration, the detected surface-landmarks from the color images can be mapped to the depth images since the face is mostly unobstructed for patient safety reasons and, hence, offer reliable information about the depth of these specific points. Comparing the depth of the different landmarks with each other enables to calculate the tilt of the entire head.

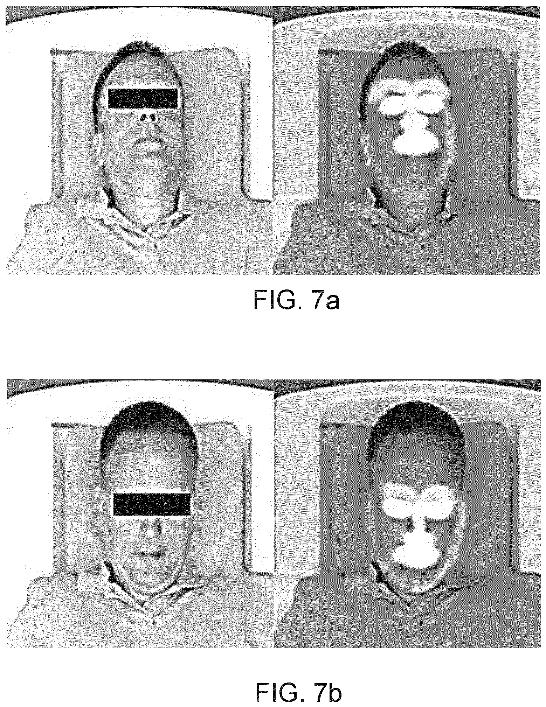

Figures 7a and 7b show images of the head of an exemplary subject acquired with an overhead camera with respective landmarks marking the eyebrows, eyes, nose, lips and the outer shape of the face. The detected landmarks are for example visualized as colored overlays (represented in black and white in Figures 7a and 7b) over the top of a grayscale version of the respective images.

Different head nodding angles are shown in Figures 7a and 7b. Different colors are for example used to represents a specific part of the face. After mapping these points to the respective depth data, the depths values of these fixed points can be compared to each other. From this depth information, a previously learned digital 3D head model can be fit to the landmarks which serves as the basis for calculating the angle of the brain plane and for deriving the optimal tilt of the head.

Once fitted to the depth data, the head model automatically allows the computation of the plane that separates the eyes from the brain. For this purpose, the same landmarks above the eyes and underneath the skull derived from the low dose scan as explained above, can be used.

To reduce or avoid ocular lens exposure, the scan angle should be parallel to a line created by the supraorbital ridge and the inner table of the posterior margin of the foramen magnum. The tilt angle of the resulting plane can be compared to the axis of the gantry to determine the correction that needs to be made. If they are already parallel, the patient and gantry can stay in their actual position. If a distinct tilt angle is detected, there are two possibilities to correct for this. In case the CT device offers to tilt the gantry, the latter can be moved in such a way that both head and gantry tilt perfectly match. If a gantry tilt is not provided, the head needs to be moved manually, e.g. by replacing the cushion or head rest.

Visual guidance on how to orient the head to match the desired tilt can be provided to the operator in real-time by means of a computer display positioned near the gantry. In an alternative setup, the head can be automatically repositioned by moving an actuator holding the head-rest or inflating or deflating an inflatable cushion under the patients head until the position is correct.

Since the face landmark detection and the fitting of the digital head model work in real-time, it is possible to check the correctness of the position instantly. Thus, the head can be moved until the system indicates a proper tilt angle. In this way, real-time guidance can be provided to the operator. When the head orientation fits to the gantry axis, the defined plane can be used to define the end of the scan range, such that the entire brain but not the eyes are part of the field of view.

In a different example, the orientation of the head is directly derived from the relative position of the landmarks in a color image, without the need for depth data. This can be achieved by e.g. learning the tilt of the brain plane from images taken with different head orientations. Due to the projective geometry of the camera, the relative positions of the face landmarks in the color image depend on the current head orientation.

The camera system thus enables a two-stage approach, starting from a coarse estimate of the head orientation derived from the optical camera images which is then subsequently refined on the basis of 3D CT surview images. The 3D surview images may instead be replaced by 2D surview images.

The camera images are used to analyze the patient head orientation. This information can in many cases be used to reposition the patient. However, optimal patient positioning is not possible in many cases, e.g. in trauma patients. Thus, based on the head orientation as estimated by the camera system, the optimal gantry tilt angle is computed and applied on the scanner. Being based on the optical camera image, this first tilt is a coarse approximation of the tilt angle to be applied for the clinical scan. As this step does not involve application of ionizing radiation, the operator can stay with the patient in the scanner room and assure the patient's safety while the gantry is being tilted.

Only after application of the gantry tilt, the 3D low dose scan is performed. Also, the scan range of the low dose scan can be limited based on the anatomy analysis from the optical camera images. This helps to avoid exposing the eyes to ionizing radiation during the low dose scan.

After the low dose scan has been acquired, the resulting 3D surview image can be analyzed to derive a more accurate estimate of the anatomy of the patient's head. Based on the 3D surview, the gantry tilt can be refined before acquisition of the clinical scan.

The provision of a camera system also enables additional functions to be implemented, to enable a better 3D low dose scan:
(i) Body shape and size can be evaluated in order to provide optimized dose level and dose modulation profile for the 3D low dose scan;
(ii) The start and end positions of the 3D low dose scan can be defined, not only the head region and tilt
(iii) An optimal couch height for the 3D low dose scan can also be derived in order to place the target anatomy as close to the iso-center as possible, for optimal image quality.

The camera system example above still makes use of an initial low dose scan. However, an alternative implementation of the camera system enables the low dose scan to be avoided altogether, by using only the optical analysis to determine the required field of view.

In an additional enhancement, AI models for the detection of foreign metal objects may be applied to the 3D low dose scan. The results can be used for altering the plane of the axial image acquisition: For the head anatomy - and other anatomies - axial slices of a patient are acquired in the clinical routine. If a foreign object is detected in such case, the availability of 3D information using 3D surview image can facilitate the generation of 3D model of foreign objects present in the field of view. Taking this information into account, the gantry can be tilted or the plane of the axial slice reconstruction can be adapted in such a way that the foreign object is avoided in the given axial field-of-view. Thus, the radiologist can look at the anatomy of interest with the least possible amount of artefacts resulting from foreign objects.

In an additional enhancement, the correctness of scan planning and patient positioning can be aggregated over a large number of scans and collected in a database. The information can be presented in a dashboard for education and training purposes that will allow technologists to gain insight into the most common positioning and scan planning issues.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a controller may be implemented by a single processor or by multiple separate processing units which may together be considered to constitute a "controller". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A CT scanner (100) for performing a brain scan, comprising:
a gantry (104, 106);
an X-ray tube (112) and detector (114) within the gantry;
a display (132); and
a controller (118), wherein the controller is configured to:
control the CT scanner to perform a low dose 3D scan with a first field of view;
analyze the results of the low dose 3D scan to derive a proposed field of view for a full dose 3D brain scan;
display a representation of the proposed field of view; and
determine a final field of view as the proposed field of view or an updated version of the proposed field of view provided by a user of the CT scanner.

2. The scanner of claim 1, wherein the first field of view includes the brain and the eyes and the final field of view excludes the eyes.

3. The scanner of claim 1 or 2, wherein the controller is configured to determine a final field of view by setting an angle for the gantry tilt.

4. The scanner of any one of claims 1 to 3, wherein the controller is configured to analyze the results of the low dose 3D scan to identify landmarks on the skull.

5. The scanner of any one of claims 1 to 4, wherein the controller is configured to derive a proposed field of view in the form of a rectangular 3D box which encloses the brain.

6. The scanner of claim 5, wherein the controller is configured to derive a rectangular 3D box as:
a box (400) with axes parallel to the CT scanner coordinate system; or
a box (402) tilted about one axis compared to a box with axes parallel to the CT scanner coordinate system; or

7. The scanner of claim 6, wherein the controller is configured to freely rotate the box in 3D space compared to a box with axes parallel to the CT scanner coordinate system to generate a diagnostic standard view representation.

8. The scanner of any one of claims 1 to 7, wherein the controller is further configured to generate images of axial superior and inferior slices for the final field of view from the low dose 3D scan.

9. The scanner of any one of claims 1 to 8, further comprising a camera system for detecting a head position and orientation of the patient relative to the CT scanner, wherein the controller is further configured to derive the first field of view from the detected head position and orientation.

10. A method of preparing a patient for a CT brain scan, comprising:
(302) controlling a CT scanner to perform a low dose 3D scan with a first field of view;
(304) analyzing the results of the low dose 3D scan to derive a proposed field of view for a full dose 3D brain scan;
(308) displaying the proposed field of view to a user of the CT system; and
(310) determining a final field of view as the proposed field of view or as an updated version of the proposed field of view provided by the user.

11. The method of claim 10, further comprising analyzing the results of the low dose 3D scan to identify landmarks on the skull, such as the supra-orbital margins of the left and right eye, the occipital bone position and a top of the skull.

12. The method of claim 10 or 11, comprising deriving a proposed field of view in the form of a rectangular 3D box which encloses the brain, and the method comprises deriving a rectangular 3D box as:
a box with axes parallel to the CT scanner coordinate system; or
a box tilted about one axis compared to a box with axes parallel to the CT scanner coordinate system.

13. The method of any one of claims 10 to 12, comprising controlling a camera system to detect a head position and orientation of the patient relative to the CT scanner and wherein the controller is further configured to derive the first field of view from the detected head position and orientation.

14. A computer program comprising computer program code which is adapted, when said program is run on a computer, to implement the method of any one of claims 10 to 13.

15. A CT scanner for performing a brain scan, comprising:
a gantry;
an X-ray tube and detector within the gantry;
a camera system for detecting a head position and orientation of the patient relative to the CT scanner;
a display; and
a controller, wherein the controller is configured to:
analyze the head position and orientation to determine a required field of view and/or patient head movement that may be required; and
output a representation of the required field of view and/or required patient head movement to a user using the display.
